# DEMANDE DE BREVET EUROPEEN

(11) **EP 1 293 774 A1**
(43) Date de publication de la demande: **19.03.2003**
(21) Numéro de dépôt: 01402351.9
(22) Date de dépôt: 12.09.2001
(51) Int. Cl.: G01N 33/00

(54) **Procede d'ajustage d'un analyseur de gaz pur**

(71) Demandeur: L'air Liquide, S.A. à Directoire et Conseil de Surveillance pour l'Etude et l'Exploitation des Procédés Georges Claude, 75321 Paris Cedex 07 (FR)
(72) Inventeur: Oztas, Cemal, 78960 Voisins le Bretonneux (FR); Guyader, Ronan, 76000 Rouen (FR); Bonet, Claude, 75014 Paris (FR)
(74) Mandataire: Mellul-Bendelac, Sylvie Lisette

(57) **Abrégé**

Un procédé d'ajustage d'un analyseur de gaz comporte au moins les étapes suivantes:
- une étape (2,4) de préparation;
- une étape (10) de vérification;
- une étape (12) d'ajustage; et
- une étape (14) de remise en service.

Application notamment aux analyseurs d'oxygène.

## Description

La présente invention concerne un procédé d'ajustage d'un analyseur de gaz.

Les analyseurs de gaz utilisés dans l'industrie permettent de déterminer la concentration ou teneur en un gaz donné dans un mélange gazeux.

Par exemple, dans l'industrie électronique le brasage des composants pour l'assemblage de cartes est réalisé sous une atmosphère inerte composée essentiellement d'azote.

Les installations correspondantes, machines de brasage à la vague ou fours de brasage par refusion, sont équipées d'analyseurs d'oxygène permettant de déterminer la teneur en oxygène de l'atmosphère à base d'azote dans laquelle s'effectue le traitement.

Les analyseurs comportent de manière classique des cellules électrochimiques qui s'usent avec le temps et peuvent être endommagées par une exposition accidentelle à l'air ambiant. De même, les circuits de transport de gaz des équipements peuvent être bouchés ou endommagés.

En conséquence, les analyseurs de gaz nécessitent une maintenance, des vérifications et des ajustages périodiques. Si cet entretien n'est pas effectué régulièrement, le taux du ou des gaz analysé(s) dans les installations n'est plus maîtrisé, ce qui aboutit à une détérioration de la qualité du traitement réalisé.

Dans certains cas, l'ajustage est fait en utilisant comme référence un gaz dont la composition n'est pas représentative des conditions de fonctionnement de l'installation. Par exemple, dans le cas de l'oxygène, on utilise comme référence l'air ambiant, dont la teneur en oxygène est mille fois supérieure à celle des équipements couramment utilisés.

Une dérive du taux d'oxygène pose en outre des problèmes de sécurité, notamment dans le cas où l'équipement de traitement fait appel à des matériaux combustibles.

La présente invention vise à remédier à ces problèmes en définissant un procédé d'ajustage d'un analyseur de gaz, permettant de garantir qualité et sécurité.

La présente invention a donc pour objet un procédé d'ajustage d'un analyseur de gaz, analyseur apte à permettre de déterminer la teneur en au moins un gaz donné (analysé) dans un mélange gazeux caractérisé en ce qu'il comporte au moins les étapes suivantes :
- une étape de préparation ;
- une étape de vérification ;
- une étape d'ajustage ; et
- une étape de remise en service.

Suivant d'autres caractéristiques de l'invention :
- les étapes de vérification et d'ajustage utilisent un gaz étalon dont la teneur en ledit gaz donné est comprise dans la plage de teneur en gaz analysé de fonctionnement de l'équipement auquel est relié l'analyseur ;
- la teneur en ledit gaz donné dudit gaz étalon est comprise entre une valeur supérieure de 10% à la teneur minimale de fonctionnement de l'équipement auquel est relié ledit analyseur et une valeur inférieure de 10% à la teneur maximale de fonctionnement ;
- ladite étape de préparation comporte une étape de préparation extérieure au site de mise en oeuvre et une étape de préparation sur le site de mise en oeuvre ;
- ladite étape de préparation extérieure comprend au moins une étape parmi les étapes suivantes :
   - une étape de prise de renseignements ;
   - une étape de vérification et de préparation de matériel ; et
   - une étape de vérification de l'aspect sécurité.
- ladite étape de préparation sur site comprend au moins une étape parmi les étapes suivantes :
   - une étape de vérification de l'aspect sécurité ; et
   - une étape d'arrêt du fonctionnement de l'équipement auquel est relié l'analyseur sur lequel est mis en oeuvre le procédé .
- le procédé comporte une étape supplémentaire de maintenance, succédant à ladite étape de préparation, et consistant à vérifier l'état d'usure de composants consommables de l'installation ;
- ladite étape de maintenance entraîne éventuellement une étape de remplacement de composants consommables, suivie par une étape de purge puis par ladite étape de vérification ;
- ladite étape de vérification entraîne éventuellement une étape de remplacement de composants consommables, suivie par une étape de purge puis de nouveau par ladite étape de vérification ;
- ladite étape de vérification comporte :
   - une première étape de raccordement dudit analyseur de gaz à un circuit d'alimentation en un gaz neutre ;
   - une étape de purge du circuit d'alimentation en gaz neutre ;
   - une étape de raccordement d'un circuit d'alimentation en gaz étalon;
   - une étape de purge du système d'alimentation en gaz étalon ; et
   - une étape d'injection dudit gaz étalon dans le circuit d'alimentation en gaz dudit analyseur de gaz .
- ladite étape d'ajustage consiste en une étape de calibrage dudit analyseur en fonction de la teneur en ledit gaz donné dudit gaz étalon ;
- les étapes de purge des circuits d'alimentation en gaz neutre et en gaz étalon comprennent une courte injection en gaz dans le système, suivie d'une purge, ces opérations étant répétées au moins cinq fois ;
- l'étape de remise en service comprend :
   - une étape de rétablissement de la connexion de l'analyseur sur le circuit d'alimentation en gaz de l'équipement ; et
   - une étape de vérification de l'étanchéité en amont et en aval dudit analyseur ;
- au moins une des étapes du procédé est suivie d'une étape de certification permettant de délivrer un procès-verbal ;
- le procédé est répété plusieurs fois avec des gaz étalon de différentes teneurs afin d'effectuer un étalonnage dudit analyseur de gaz ;
- ledit analyseur de gaz est un analyseur d'oxygène, relié à un équipement industriel d'assemblage de cartes électroniques ;
- ledit gaz étalon utilisé dans ledit procédé, a une teneur en oxygène inférieure ou égale à 10000 ppm, de préférence inférieure ou égale à 5000 ppm et plus préférentiellement inférieure ou égale à 1000 ppm;
- le procédé est mis en oeuvre par du personnel titulaire d'une habilitation délivrée à l'issue d'une formation et/ou d'un entraînement.

L'invention sera mieux comprise à la lecture de la description qui va suivre, donnée uniquement à titre d'exemple et faite en se référant aux dessins annexés, sur lesquels :
- la Fig.1 est un organigramme général d'un procédé d'ajustage selon l'invention ; et
- les Figs. 2 et 3 sont des schémas de principe d'une installation lors des phases de vérification et d'ajustage du procédé selon l'invention.

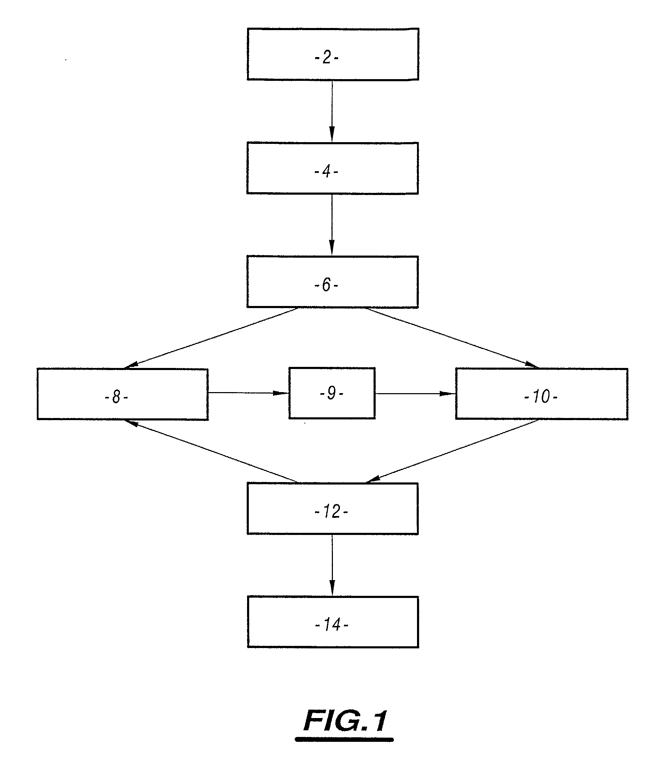

La figure 1 représente un organigramme d'un procédé d'ajustage d'un analyseur de gaz selon l'invention.

Ce procédé comprend une première étape 2 de préparation hors site qui consiste en une vérification de l'aspect logistique.

En particulier, cette étape 2 consiste à s'assurer de la disponibilité des intervenants et à prendre connaissance des accords et contrats existants.

Elle consiste également à définir puis à se munir du matériel nécessaire à la mise en oeuvre du procédé, tel que par exemple :
- véhicule adapté pour le transport des bouteilles de gaz ;
- bouteille de gaz étalon ;
- détendeur de précision ;
- détendeur spécifique à l'équipement sur lequel le procédé va être mis en oeuvre ;
- raccord de canalisation non poreux au gaz à analyser ;
- débitmètre ;
- dispositif de filtrage ;
- matériel et composants consommables de rechange en fonction des contrats et accords ;
- dispositif d'échangeur gazeux.

Cette étape 2 de préparation hors site consiste également à se renseigner sur le plan de prévention du lieu de mise en oeuvre du procédé et éventuellement de se munir de matériel de sécurité complémentaire.

Le procédé comprend ensuite une étape 4 de préparation effectuée sur le site de l'installation concernée.

Par exemple, l'étape 4 de préparation sur site consiste à prendre connaissance des spécifications du site de mise en oeuvre du procédé, et notamment des aspects sécurité et prévention.

Cette étape 4 fait intervenir le personnel mettant en oeuvre le procédé d'ajustage, ainsi que le personnel ayant la maîtrise du site.

Le procédé comporte ensuite une étape 6 de maintenance consistant à vérifier l'état de l'analyseur de gaz et notamment l'état d'usure des composants consommables.

Par exemple, en fonction de sa nature, on peut vérifier l'état d'un filtre disposé en amont de, ou intégré dans l'analyseur, ainsi que le niveau d'utilisation de l'électrolyte dans le cas où l'analyseur est pourvu d'une cellule à électrolyte etc...

Cette étape 6 peut comporter de plus un examen du circuit de transport de gaz, basé sur une analyse visuelle ainsi que sur la mesure faite avec des moyens de mesure de débit, tels qu'un débitmètre.

L'étape 6 de maintenance peut déboucher sur une étape 8 de remplacement des consommables de l'analyseur de gaz, en fonction des besoins et/ou des accords et/ou des commandes.

Le remplacement de consommable qui est effectué de manière classique, est suivi d'une étape 9 de purge du système qui peut durer plusieurs heures au besoin.

Une fois l'étape 9 de purge effectuée, on passe à une étape 10 de vérification.

Dans le cas où lors de l'étape 6 de maintenance, il s'est révélé inutile de se rendre à l'étape 8 de remplacement de consommable, on peut passer directement de l'étape 6 de maintenance à l'étape 10 de vérification.

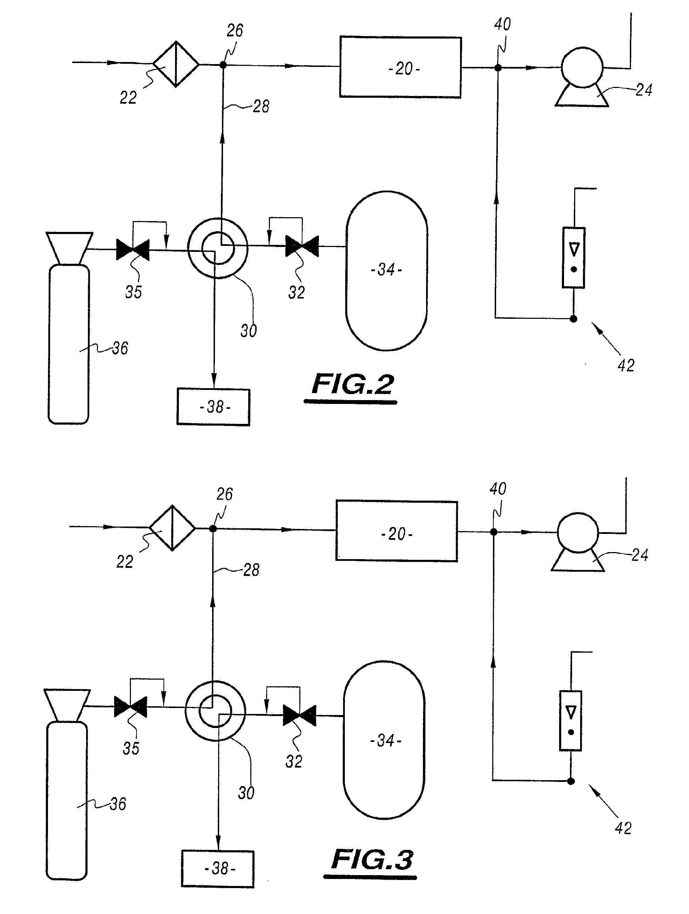

Cette étape 10 de vérification, décrite plus loin en référence aux figures 2 et 3, prévoit l'utilisation d'un gaz étalon et consiste à vérifier que ce gaz étalon est convenablement diffusé dans les circuits de l'analyseur.

La teneur en gaz à analyser du gaz étalon doit être connue et certifiée. De plus, cette teneur en gaz à analyser doit se situer dans la plage d'utilisation de l'équipement auquel est relié l'analyseur de gaz.

Par exemple, dans le cas d'un analyseur d'oxygène, utilisation d'un gaz étalon composé d'une matrice d'azote comportant de l'oxygène à hauteur de 50 moles d'oxygène par millions de moles d'azote (ppm moles).

L'incertitude d'étalonnage d'un tel mélange est, par exemple, inférieure à 1%.

Dans tous les cas, il convient de s'assurer que la teneur en gaz mesurée, dans cet exemple la teneur en oxygène, est comprise dans la plage de fonctionnement de l'équipement auquel il est connecté.

Avantageusement, la teneur en gaz à analyser est comprise entre une valeur supérieure de 10% à la teneur minimale en gaz à analyser du système et une valeur inférieure de 10% à la teneur maximale.

Suite à l'étape 10 de vérification, on procède à l'étape 12 d'ajustage de l'analyseur.

L'utilisation d'un gaz étalon de concentration connue et dont la diffusion dans le système a été vérifiée lors de l'étape 10 de vérification, permet d'effectuer un calibrage des outils de mesure de l'analyseur de gaz lors de cette étape 12 d'ajustage.

En effet, la teneur en gaz mesurée par l'analyseur correspond au gaz à analyser introduit dans le système par le gaz étalon. On peut donc ajuster l'analyseur de manière à ce que la valeur qu'il affiche corresponde à la teneur en gaz à analyser du gaz étalon.

Les spécificités de l'étape 12 d'ajustage tiennent aux spécificités de l'analyseur de gaz sur lequel le procédé est mis en oeuvre.

Par exemple, cette étape 12 d'ajustage consiste à régler un potentiomètre à l'aide d'un afficheur ou encore à calibrer un afficheur numérique en sélectionnant son échelle et en introduisant un décalage.

Lors de cette étape 12 d'ajustage, des problèmes rencontrés peuvent induire la nécessité de procéder à un remplacement de consommable qui n'était pas apparu nécessaire lors de l'étape 6 de maintenance.

Dans ce cas, on procède à l'étape 8 de remplacement des consommables pour se rendre, après l'étape 9 de purge, à l'étape 10 de vérification et revenir ensuite à l'étape 12 d'ajustage.

Une fois l'étape 12 d'ajustage convenablement effectuée, on procède à une étape 14 de remise en service consistant à reconnecter l'analyseur de gaz sur l'équipement de traitement selon la configuration fonctionnelle et en s'assurant de l'étanchéité du système afin de procéder à une remise en service de manière classique.

Eventuellement, l'étape 14 comporte une étape de rédaction d'un procès-verbal d'entretien et d'ajustage de l'analyseur.

En référence aux figures 2 et 3, on va maintenant exposer le détail de l'étape 10 de vérification à l'aide d'un gaz étalon dans le cas de l'exemple d'un analyseur d'oxygène connecté à un circuit d'alimentation en azote.

Sur la figure 2, on a représenté schématiquement un analyseur d'oxygène 20 placé en série sur un circuit d'alimentation en azote et relié en amont à un filtre 22 et en aval, à une pompe 24.

A l'aide d'un raccord 26 à double bagues, on connecte sur le circuit d'alimentation en gaz de l'analyseur 20 les éléments nécessaires à l'étape 10 de vérification à l'aide du gaz étalon.

Ce raccordement est fait à l'aide d'un tube 28 non poreux à l'oxygène.

On raccorde au tube 28 non poreux à l'oxygène, une vanne 30 à quatre voies. Celle-ci est reliée par l'intermédiaire d'un détendeur 32 à un réservoir 34 d'azote de purge et par l'intermédiaire d'un détendeur 35, à une bouteille 36 de gaz étalon.

Le réservoir 34 d'azote de purge peut être directement le réseau d'alimentation en azote ou une bouteille spécialement destinée à cette fonction.

La vanne 30 à quatre voies comporte une mise à l'air libre 38.

Lors de la connexion des différents éléments de canalisation, il est nécessaire de s'assurer que les différents systèmes de purge et d'alimentation sont fermés, éventuellement de boucher les systèmes de mise à l'air et de purge et enfin d'éviter au maximum les volumes morts de gaz, notamment dans les éventuelles vannes d'isolement.

Il convient également de mettre en place un circuit d'échappement du gaz en aval de l'analyseur 20.

Ce circuit doit être le plus direct possible et le cas échéant contourner les éléments de fonctionnement tels que la pompe 24 à l'aide d'une dérivation introduite via un raccord 40 à double bagues. On place sur ce circuit d'échappement un débitmètre 42 qui permet de vérifier le débit d'écoulement du gaz au travers du circuit d'échappement et de l'analyseur 20.

Dans le cas où l'on ne peut pas contourner la pompe 24, il convient d'augmenter le débit du gaz entrant dans l'analyseur et de régler la pompe 24 afin que sa résistance à la circulation du gaz soit minimale.

L'étape 10 de vérification commence par la mise en service du matériel décrit. Notamment, l'installation de la bouteille 36 de gaz étalon et celle du réservoir d'azote 34 nécessitent la mise en service et la purge des détendeurs 32 et 35.

Cette étape de purge nécessite des vérifications optionnelles préalables afin de s'assurer de la bonne connexion des éléments et la répétition d'un cycle de mise en pression et de vidange.

Pour ce faire, à l'aide de la vanne 30, on relie la bouteille 34 d'azote de purge au circuit d'alimentation de l'analyseur 20 au travers du raccord 26.

Le cycle de mise en pression et de vidange de l'air, commence par l'ouverture puis la fermeture du robinet de la bouteille 34 d'azote de purge afin de commencer à insuffler de l'azote dans les conduites.

Il convient d'attendre pendant un délai d'au moins une minute, avant de procéder à la purge, de manière à obtenir une bonne dilution de l'air dans le gaz de purge.

On effectue ensuite la purge du détendeur 32 situé entre le réservoir 34 d'azote de purge et la vanne 30 à quatre voies sans toutefois laisser la pression diminuer jusqu'à atteindre la pression atmosphérique.

Ce cycle sera renouvelé par exemple cinq fois afin d'être sûr d'avoir expulsé tout l'air de la conduite 28 et du détendeur 32.

On effectue ensuite une purge du système complet en ouvrant le circuit d'alimentation à partir de la bouteille d'azote 34 et en laissant le gaz de purge s'écouler librement.

Dans le cas où l'on dispose du débitmètre 32, on vérifie le débit du gaz s'écoulant au travers de l'analyseur 20, et si nécessaire, on le règle à l'aide du détendeur 32 afin d'atteindre le débit maximal de fonctionnement de l'analyseur 20 lors de la purge du système.

Une éventuelle variation de ce débit peut permettre de déceler un défaut de canalisation qu'il convient de réparer avant de continuer.

Une fois que le système est stabilisé, à savoir que le débit mesuré sur le débitmètre 42 est stable et/ou que la valeur affichée par l'analyseur 20 est stable, on peut procéder au raccordement de la bouteille 36 du gaz étalon.

Pendant que l'on effectue l'installation de la bouteille 36 de gaz étalon, on maintient l'analyseur 20 alimenté à partir de la bouteille 34 d'azote de purge.

L'installation commence par une étape de purge du détendeur 35 situé entre la bouteille 36 de gaz étalon et la vanne à quatre voies 30.

Ainsi que cela a été dit précédemment pour la purge du détendeur 32, cette étape de purge est un cycle consistant à ouvrir puis refermer aussitôt le robinet d'alimentation de la bouteille, à attendre pendant une période d'au moins une minute de manière à obtenir une bonne dilution de l'air dans le gaz, puis à purger, sans atteindre la pression atmosphérique, le détendeur 35.

Par exemple, on renouvelle ce cycle cinq fois.

Afin de régler le bon débit de gaz étalon, on utilise soit un débitmètre intégré dans le détendeur 35, soit un débitmètre supplémentaire non représenté que l'on branche à la mise à l'air libre 38 de la vanne 30 à quatre voies.

On règle le débit de manière à l'adapter à l'analyseur 20 en fonction des caractéristiques techniques de celui-ci.

Pendant quelques minutes, on laisse le gaz étalon s'échapper à l'air libre de manière à purger les canalisations entre la bouteille étalon 36 et la vanne 30.

On alimente ensuite l'analyseur 20 en gaz étalon.

Ainsi que cela est représenté en référence à la figure 3, on bascule la vanne 30 à quatre voies de manière à connecter la bouteille 36 de gaz étalon au circuit d'alimentation de l'analyseur 20.

On peut alors certifier que l'oxygène mesuré par l'analyseur 20 dans le mélange de gaz circulant est uniquement l'oxygène insufflé à partir de la bouteille 36 de gaz étalon. La différence entre la teneur en oxygène du gaz étalon contenu dans la bouteille étalon 36 et la teneur en oxygène mesurée par l'analyseur 20, est due à un mauvais réglage ou à une usure de l'analyseur et sera corrigée lors de l'étape d'ajustage 12.

Il apparaît donc que ce procédé permet de s'assurer que l'ajustage de l'analyseur est fait dans des conditions optimales.

La mise en oeuvre répétée de cette procédure avec des gaz étalon de différentes teneurs permet d'effectuer l'étalonnage d'un analyseur donné.

Bien entendu, une telle procédure, notamment au niveau de la connexion des différents éléments nécessaires à sa mise en oeuvre, doit être adaptée et prendre en compte les différences entre les installations.

En outre, chaque étape d'un tel procédé peut être sanctionnée par un procès-verbal.

Afin de permettre la bonne mise en oeuvre de ce procédé, il convient que le personnel soit convenablement entraîné. Le procédé de l'invention fait donc préférentiellement l'objet d'une habilitation délivrée à du personnel ayant reçu une formation et/ou un entraînement appropriés.

## Revendications

1. Procédé d'ajustage d'un analyseur de gaz (20), analyseur apte à permettre de déterminer la teneur en au moins un gaz donné dans un mélange gazeux, **caractérisé en ce qu'**il comporte au moins les étapes suivantes :
- une étape (2,4) de préparation ;
- une étape (10) de vérification ;
- une étape (12) d'ajustage ; et
- une étape (14) de remise en service.

2. Procédé selon la revendication 1, **caractérisé en ce que** les étapes de vérification (10) et d'ajustage (12) utilisent un gaz étalon dont la teneur en ledit gaz donné est comprise dans la plage de teneur en ledit gaz donné de fonctionnement de l'équipement auquel est relié l'analyseur (20).

3. Procédé selon la revendication 2, **caractérisé en ce que** la teneur en ledit gaz donné dudit gaz étalon est comprise entre une valeur supérieure de 10% à la teneur minimale de fonctionnement de l'équipement auquel est relié ledit analyseur de gaz (20) et une valeur inférieure de 10% à la teneur maximale de fonctionnement.

4. Procédé selon l'une quelconque des revendications 1 à 3, **caractérisé en ce que** ladite étape de préparation (2,4) comporte une étape (2) de préparation extérieure au site de mise en oeuvre et une étape (4) de préparation sur le site de mise en oeuvre.

5. Procédé selon la revendication 4, **caractérisé en ce que** ladite étape (2) de préparation extérieure comprend au moins une étape parmi les étapes suivantes :
- une étape de prise de renseignements ;
- une étape de vérification et de préparation de matériel ; et
- une étape de vérification de l'aspect sécurité.

6. Procédé selon l'une des revendications 4 ou 5, **caractérisé en ce que** ladite étape (4) de préparation sur site comprend au moins une étape parmi les étapes suivantes :
- une étape de vérification de l'aspect sécurité ; et
- une étape d'arrêt du fonctionnement de l'équipement auquel est relié l'analyseur sur lequel est mis en oeuvre le procédé.

7. Procédé selon l'une quelconque des revendications 1 à 6, **caractérisé en ce qu'**il comporte une étape supplémentaire (6) de maintenance, succédant à ladite étape (2,4) de préparation, et consistant à vérifier l'état d'usure de composants consommables de l'installation.

8. Procédé selon la revendication 7, **caractérisé en ce que** ladite étape (6) de maintenance entraîne éventuellement une étape (8) de remplacement de composants consommables, suivie par une étape (9) de purge puis par ladite étape (10) de vérification.

9. Procédé selon l'une quelconque des revendications 7 ou 8, **caractérisé en ce que** ladite étape (10) de vérification entraîne éventuellement une étape de remplacement de composants consommables (8), suivie par une étape (9) de purge puis de nouveau par ladite étape (10) de vérification.

10. Procédé selon l'une quelconque des revendications 2 à 9, **caractérisé en ce que** ladite étape (10) de vérification comporte :
- une première étape de raccordement dudit analyseur de gaz (20) à un circuit d'alimentation en un gaz neutre (30,32,34) ;
- une étape de purge du circuit d'alimentation en gaz neutre (30,32, 34);
- une étape de raccordement d'un circuit d'alimentation en gaz étalon (30,35,36) ;
- une étape de purge du système d'alimentation en gaz étalon ; et
- une étape d'injection dudit gaz étalon dans le circuit d'alimentation en gaz dudit analyseur de gaz (20).

11. Procédé selon la revendication 10, **caractérisé en ce que** ladite étape (12) d'ajustage consiste en une étape de calibrage dudit analyseur (20) en fonction de la teneur en ledit gaz donné dudit gaz étalon .

12. Procédé selon la revendication 10, **caractérisé en ce que** les étapes de purge des circuits d'alimentation en gaz neutre (30, 32, 34) et en gaz étalon (30, 35, 36) comprennent une courte injection en gaz dans le système, suivie d'une purge, ces opérations étant répétées au moins cinq fois.

13. Procédé selon l'une quelconque des revendications 1 à 12, **caractérisé en ce que** l'étape de remise en service (14), comprend :
- une étape de rétablissement de la connexion de l'analyseur (20) sur le circuit d'alimentation en gaz de l'équipement ; et
- une étape de vérification de l'étanchéité en amont et en aval dudit analyseur (20).

14. Procédé selon l'une quelconque des revendications 1 à 13, **caractérisé en ce qu'**au moins une des étapes du procédé (2,4,6,8,10,12, 14) est suivie d'une étape de certification permettant de délivrer un procès-verbal.

15. Procédé selon l'une quelconque des revendications 1 à 14, **caractérisé en ce qu'**il est répété plusieurs fois avec des gaz étalon de différentes teneurs afin d'effectuer un étalonnage dudit analyseur de gaz (20).

16. Procédé selon l'une quelconque des revendications 2 à 15, **caractérisé en ce que** ledit analyseur de gaz (20) est un analyseur d'oxygène, relié à un équipement industriel d'assemblage de cartes électroniques.

17. Procédé selon la revendication 16, **caractérisé en ce que** ledit gaz étalon utilisé dans ledit procédé, a une teneur en inférieure ou égale à 10000 ppm, de préférence inférieure ou égale à 5000 ppm et plus préférentiellement inférieure ou égale à 1000 ppm.

18. Procédé selon l'une quelconque des revendications 1 à 17, **caractérisé en ce qu'**il est mis en oeuvre par du personnel titulaire d'une habilitation délivrée à l'issue d'une formation et/ou d'un entraînement.
